# EUROPÄISCHE PATENTSCHRIFT

(11) **EP 2 991 966 B1**
(45) Veröffentlichungstag und Bekanntmachung des Hinweises auf die Patenterteilung: **12.07.2017**
(21) Anmeldenummer: 14719020.1
(22) Anmeldetag: 24.04.2014
(51) Int. Cl.: C07C 201/08

(54) **VERFAHREN ZUR HERSTELLUNG VON NITROBENZOL DURCH ADIABATE NITRIERUNG**
METHOD FOR MANUFACTURING NITROBENZENE THROUGH ADIABATIC NITRATION
PROCEDE DESTINE À LA FABRICATION DE BENZOL NITRIQUE PAR NITRIFICATION D'ADIABATE

(30) Priorität: 29.04.2013 EP 13165822
(43) Veröffentlichungstag der Anmeldung: 09.03.2016
(73) Patentinhaber: Covestro Deutschland AG, 51373 Leverkusen (DE)
(72) Erfinder: MAIRATA, Antoni, 40549 Düsseldorf (DE); KNAUF, Thomas, 41542 Dormagen (DE)
(74) Vertreter: Levpat
(86) Internationale Anmeldenummer: PCT/EP2014/058345
(87) Internationale Veröffentlichungsnummer: WO 2014/177450

(56) Entgegenhaltungen:
- EP-A1- 2 070 907

## Beschreibung

Die vorliegende Erfindung betrifft ein kontinuierlich betriebenes adiabates Verfahren zur Herstellung von Nitrobenzol durch Nitrierung von Benzol mit Salpetersäure und Schwefelsäure, bei dem die nach erfolgter Nitrierung und Abtrennung des rohen Nitrobenzols von der wässrigen Phase erhaltene verdünnte Schwefelsäure zum Zwecke der Wiederverwendung in der Nitrierung aufkonzentriert und nach ihrer Aufkonzentrierung mindestens eine Minute, bevor sie wieder mit frischer Salpetersäure in Kontakt tritt, so mit einem Oxidationsmittel versetzt wird, dass sich eine Konzentration des Oxidationsmittels von 10 ppm bis 5.000 ppm, bezogen auf die Gesamtmasse der in die Nitrierung zurückzuführenden aufkonzentrierten Schwefelsäure, einstellt.

Nitrobenzol ist ein wichtiges Zwischenprodukt der chemischen Industrie, das insbesondere zur Herstellung von Anilin und damit auch zur Herstellung der Di- und Polyisocyanate der Diphenylmethanreihe und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Mischsäure*)*.* Ein solches Verfahren wurde erstmals in US 2,256,999 beansprucht und ist in heutigen Ausführungsformen beispielsweise in EP 0 436 443 B1, EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen. Die Ausgangsstoffe Benzol und Salpetersäure werden in einem großen Überschuss an Schwefelsäure zur Reaktion gebracht, welche die frei werdende Reaktionswärme und das bei der Reaktion gebildete Wasser aufnimmt.

Die Reaktionsführung erfolgt im Allgemeinen so, dass die Salpetersäure und Schwefelsäure zur sogenannten Nitriersäure (auch Mischsäure genannt) vereint werden. Benzol wird in diese Nitriersäure hinein dosiert. Die Reaktionsprodukte sind im Wesentlichen Wasser und Nitrobenzol. In der Nitrierreaktion wird Benzol bezogen auf die molare Salpetersäuremenge mindestens in stöchiometrischer Menge, aber bevorzugt im 2 %igem bis 10 %igem Überschuss eingesetzt. Das im Wesentlichen salpetersäurefreie Reaktionsgemisch, das nach der Reaktionszone erhalten wird, wird einem Phasentrennapparat zugeführt, in dem sich zwei Phasen bilden, wobei die erste als Rohnitrobenzol bezeichnet wird und im Wesentlichen aus Nitrobenzol, Benzol und einer im Nitrobenzol gelösten Menge an Schwefelsäure und Wasser besteht, und die zweite als Kreislaufsäure bezeichnete im Wesentlichen aus Wasser, Schwefelsäure und in der Schwefelsäure gelöstem Nitrobenzol besteht. Die im Phasentrennapparat abgetrennte Kreislaufsäure wird, wie in US 5 313 009 beschrieben, in einen Apparat zur Blitzverdampfung des Wassers eingebracht, in dem durch Anlegen eines Unterdrucks und unter Ausnutzung der hohen Temperatur der Kreislaufsäure, die durch die adiabate Reaktionsführung erreicht wurde, Wasser aus der Kreislaufsäure verdampft wird, so dass eine konzentrierte Schwefelsäure erhalten wird, deren Konzentration im Wesentlichen der Konzentration vor der Reaktionszone entspricht. Nach der Schwefelsäureaufkonzentrierung wird die so erhaltene Schwefelsäure ohne weitere Behandlung in die Reaktion gefahren.

Die Güte eines adiabaten Verfahrens zur Nitrierung aromatischer Kohlenwasserstoffe ist einerseits definiert durch den Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Das Bestreben bei der Herstellung von Nitrobenzol ist es, den Gehalt an Dinitrobenzol und an Nitrophenolen, insbesondere des als brisant einzustufenden Trinitrophenols (Pikrinsäure), zu minimieren. Andererseits ist die Güte eines adiabaten Verfahrens dadurch definiert, das Nitrobenzol ohne technischen Produktionsausfall hergestellt werden kann.

Durch die Rückführung der Schwefelsäure entsteht ein Schwefelsäurekreislauf, der aus Reaktionszone, Phasentrennapparat, Verdampfer, Pufferbehälter und verbindenden Leitungen besteht. In EP 2 070 907 A1 ist beschrieben, dass in der Schwefelsäure Metallionen vorhanden sein können, die zusammen mit Sulfat in Schwefelsäure schwer lösliche Metallsulfate bilden. Diese Metalle umfassen die Elemente Al, Ca, Cr, Mn, Fe, Co, Ni, Cu, Sr, Cd und Ba, insbesondere Ca und Fe. Übersteigt die Konzentration dieser schwerlösliche Metallsulfate bildenden Metallionen die Löslichkeitsgrenze, so fallen Metallsulfate in Form eines Feststoffes in der Schwefelsäure aus und werden als Feststoffe mit der Schwefelsäure im Kreislauf mitgeführt, bis sie sich an einer Fläche oder einer Engstelle absetzen und ansammeln.

Es ist ebenfalls ein bekanntes Phänomen, dass die Löslichkeitsgrenze der schwerlösliche Metallsulfate bildenden Metallionen stark von der Temperatur der Lösung, also der Temperatur der Schwefelsäure, abhängt. So lösen sich in kalter Schwefelsäure weniger Metallionen als in heißer und demzufolge fallen in kalter Schwefelsäure oder an Stellen, an denen Schwefelsäure abgekühlt wird, wie es z. B. in Wärmeaustauschern der Fall ist, besonders leicht Metallsulfate als Feststoff an. Dieser Anfall von Feststoffen in Wärmeaustauschern ist als problematisch anzusehen, da er zu einer Belegung der Oberfläche des Wärmeaustauschers und damit zu einer Verschlechterung des Wärmeübergangskoeffizienten führt und auch durch die Verringerung des freien Querschnitts der Leitungen im Wärmetauscher die mögliche Durchflussmenge durch Denselben begrenzt.

EP 2 070 907 A1 offenbart nun, dass eine Reinigung von Wärmeaustauschern und Schwefelsäure führenden Leitungen von ausgefallenen festen Metallsulfaten nicht mehr nötig ist, wenn bei der Nitrierung des Benzols durch eine Schwefel- und Salpetersäure-haltige Mischsäure die durch Blitzverdampfung von Wasser wiedererhaltene Schwefelsäure nicht vollständig als Kreislaufsäure in die Reaktionszone zurückgeführt wird, sondern teilweise ausgeschleust und durch frische, Metallionen-arme Schwefelsäure ersetzt wird. Ein periodisches Spülen der Apparate, wie z. B. all jener Wärmeaustauscher, die Kreislaufsäure führen, um die aus der konzentrierten Schwefelsäure kristallisierten Metallsulfate zu entfernen (DE 340 97 17 C2), kann damit entfallen.

Entgegen der in EP 2 070 907 A1 offenbarten Lösung der Vermeidung von Niederschlägen in Wärmeaustauschern und Rohrleitungen einer Nitrieranlage wurden bei kontinuierlichem Betrieb einer, wie in EP 2 070 907 A1 beschriebenen, Nitrieranlage schwarze Kohlenstoff-haltige Niederschläge gefunden, die sich im Phasentrennapparat des Rohprodukts, im Rohnitrobenzolkühler und in den benachbarten Rohrleitungen absetzten.

Es bestand daher ein Bedarf an einer weiteren Verbesserung des Verfahrens zur Herstellung von Nitrobenzol. Insbesondere sollte ein reibungsloses Betreiben der kontinuierlichen, adiabaten Nitrierung von Benzol gewährleistet werden, d. h., insbesondere die Entstehung von störenden Niederschlägen, die im Extremfall dazu führen können, dass der kontinuierliche Prozess für Reinigungszwecke abgefahren werden muss, sollte vermieden werden.

Diesem Bedarf Rechnung tragend ist ein Gegenstand der vorliegenden Erfindung ein kontinuierlich betriebenes adiabates Verfahren zur Herstellung von Nitrobenzol durch Nitrierung von Benzol, bei dem
a) ein Benzol-haltiger Strom (a.1) in einem Reaktor mit Schwefelsäure (a.2) und Salpetersäure (a.3) unter adiabaten Bedingungen umgesetzt wird, wobei Benzol in einem stöchiometrischen Überschuss bezogen auf Salpetersäure (a.3) von bevorzugt 2,0 % bis 20 %, besonders bevorzugt von 5,0 % bis 10 % der Theorie eingesetzt wird,
b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, wobei die Phase (c.1) in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, bevorzugt durch Waschen mit wässrigen Medien und sich daran anschließender Rektifikation, wobei überschüssiges Benzol, das als sogenanntes Rückbenzol in der Nitrierung Wiederverwendung findet, und Wasser vom Endprodukt abgetrennt werden,
wobei
der aufkonzentrierten wässrigen, Schwefelsäure-haltigen Phase (c.1) mindestens 1 Minute, bevorzugt 1 Minute bis 10 Minuten, besonders bevorzugt 3 Minuten bis 7 Minuten, bevor diese mit dem Salpetersäurestrom (a.3) in Kontakt tritt, ein Oxidationsmittel (c.2) ausgewählt aus Salpetersäure (c.2.1), salpetrige Säure (c.2.2) und/oder Nitrosylsulfonsäure (c.2.3) in einem Maß zugesetzt wird, dass sich eine Konzentration an Oxidationsmittel (c.2) von 10 ppm bis 5.000 ppm, bevorzugt 50 ppm bis 2.000 ppm und besonders bevorzugt 100 ppm bis 1.000 ppm, jeweils bezogen auf die Gesamtmasse der in Schritt a) zurückzuführenden aufkonzentrierten, wässrigen, Schwefelsäure-haltigen Phase (c.1), einstellt.

Überraschend wurde nämlich gefunden, dass die Zugabe geringer Mengen eines Oxidationsmittels ausgewählt aus Salpetersäure (c.2.1), salpetrige Säure (c.2.2) und/oder Nitrosylsulfonsäure (c.2.3) zur Kreislaufschwefelsäure während der Rückführung zur Reaktion die Bildung der zuvor genannten Niederschläge vermindert bis völlig verhindert.

Der *auf Salpetersäure bezogene Beuzolüberschuss* von 2,0 % bis 20 %, bevorzugt von 5,0 % bis 10 % der Theorie, bezieht sich auf das molare Verhältnis von Benzol und Salpetersäure. Theoretisch reagiert ein Mol Salpetersäure mit einem Mol Benzol zu einem Mol Nitrobenzol.

Die oben genannten *Kouzentrationen an Oxidationsmittel (c.2) in ppm* beziehen sich auf das reine Oxidationsmittel, im Falle von beispielsweise Salpetersäure (c.2.1) also auf hypothetische reine HNO₃ und nicht auf die wässrige Lösung. Das Oxidationsmittel (c.2) kann erforderlichenfalls auch *in situ* erzeugt werden; bspw. durch Zugabe von Natriumnitrit zur Schwefelsäure-haltigen Phase (c.1) (Bildung von salpetriger Säure (c.2.2)). Auch in diesen Fällen bezieht sich die *Konzentration an Oxidationsmittel (c.2) in ppm* auf das reine Oxidationsmittel, also im genannten Beispiel auf hypothetisch reines HNO₂.

Nachstehend werden die einzelnen Schritte der Erfindung detailliert erläutert. Verschiedene Ausführungsformen sind dabei beliebig miteinander kombinierbar, solange sich für den Fachmann aus dem Kontext nicht eindeutig das Gegenteil ergibt.

**Schritt a)** kann grundsätzlich nach allen aus dem Stand der Technik bekannten adiabaten Nitrierverfahren durchgeführt werden. Für die Ausführung dieses Schritts des erfindungsgemäßen Verfahrens wird bevorzugt ein Rohrreaktor eingesetzt, in dem mehrere Dispergierelemente über die Länge des Reaktors verteilt angeordnet sind, die eine intensive Dispergierung und Durchmischung von Benzol, Salpetersäure und Schwefelsäure gewährleisten. Ein solcher Reaktor, sowie die Form einsetzbarer Dispergierelemente, sind beispielsweise in EP 0708 076 B1 (Figur 2) und EP 1 291 078 A2 (Figur 1) beschrieben. Bevorzugt wird Schritt a) in einer Verfahrensführung ausgeführt, wie sie in DE 10 2008 048 713 A1, insbesondere Absatz [0024], beschrieben ist. Bevorzugt ist dabei, zuerst die Salpetersäure (a.3) und anschließend das Benzol (a.1) in die Schwefelsäure (a.2) einzudosieren. Die Verweilzeit zwischen der Zugabe von Salpetersäure in die Kreislaufschwefelsäure und der Zugabe von Benzol in das Nitriersäuregemisch am Eintritt des Nitrierreaktors beträgt wenige Sekunden.

Die Phasentrennung in **Schritt b)** erfolgt ebenfalls nach aus dem Stand der Technik an sich bekannten Verfahren in einem dem Fachmann bekannten Trennbehälter. Die wässrige Phase (b.1) enthält im Wesentlichen (infolge der Bildung von Reaktionswasser und durch das Einschleppen von Wasser in die Reaktion aus der eingesetzten Salpetersäure) verdünnte Schwefelsäure neben anorganischen Verunreinigungen, die organische Phase (b.2) enthält im Wesentlichen Nitrobenzol neben überschüssigem Benzol und organischen Verunreinigungen.

Die Aufkonzentrierung der wässrigen Phase (b.1) in **Schritt c)** erfolgt grundsätzlich wie aus dem Stand der Technik bekannt. Die Schwefelsäure in der wässrigen Phase wird in einem Entspannungsverdampfer (auch als Blitzverdampfer bezeichnet) aufkonzentriert, indem Wasser in einen Bereich reduzierten Druckes hinein verdampft wird. Bei richtiger Wahl der Reaktionsbedingungen in der adiabaten Nitrierung von Benzol mit Mischsäure erzielt man mit der Reaktionswärme der exothermen Reaktion eine so starke Erhitzung der Schwefelsäure enthaltenden wässrigen Phase (b.1), dass im Entspannungsverdampfer gleichzeitig wieder die Konzentration und Temperatur der Schwefelsäure enthaltenden wässrigen Phase eingestellt werden kann, die diese vor der Reaktion mit Benzol und Salpetersäure bei Eintritt in den Reaktorraum hatte, d. h., (c.1) entspricht hinsichtlich Temperatur und Konzentration (a.2). Dies ist beschrieben in EP 2 354 117 A1, insbesondere Absatz [0045].

Die Aufarbeitung der organischen Phase (b.2) in **Schritt d)** erfolgt grundsätzlich wie aus dem Stand der Technik bekannt. Eine bevorzugte Verfahrensweise wird nachstehend geschildert:

Die organische Phase (b.2), die üblicherweise noch Spuren an Säure enthält, wird in einer bis zwei Wäschen, bevorzugt einer Wäsche, mit einer wässrigen Waschflüssigkeit gewaschen und dann von der sauren wässrigen Phase durch Phasentrennung getrennt; bei mehreren Wäschen nach jeder einzelnen Wäsche (Schritt d(i)). Bei diesem Vorgang werden die Säurereste, die das rohe Nitrobenzol (b.2) enthält, ausgewaschen; daher wird dieser Verfahrensschritt auch als *saure Wäsche* bezeichnet. Dieser Schritt ist aus dem Stand der Technik hinlänglich bekannt und wird daher hier nur kurz umrissen. Bevorzugt werden zur Durchführung dieser sauren Wäsche im Betrieb anfallende wässrige Ströme rezyklisiert.

Die so erhaltene organische Phase wird anschließend in einer bis zwei, bevorzugt einer alkalischen Wäsche(n) mit einer wässrigen Lösung einer Base, bevorzugt ausgewählt aus Natriumhydroxid, Natriumcarbonat oder Natriumhydrogencarbonat, gewaschen und anschließend von dem alkalischen Waschwasser durch Phasentrennung getrennt; bei mehreren Wäschen nach jeder einzelnen Wäsche (Schritt d(ii)). Besonders bevorzugt wird als wässrige Baselösung Natronlauge eingesetzt. Dieser Schritt ist aus dem Stand der Technik hinlänglich bekannt und wird daher hier nur kurz umrissen. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt d(i) nicht vollständig entfernte Säurereste) weitgehend bis vollständig, bevorzugt vollständig, in der alkalischen Wäsche neutralisiert werden. Die anschließende Aufarbeitung des alkalischen Abwassers kann nach den Verfahren des Standes der Technik, z. B. gemäß EP 1 593 654 A1 und EP 1 132 347 A2 erfolgen.

Die so erhaltene organische Phase wird schließlich in mindestens einer, bevorzugt zwei bis vier, besonders bevorzugt zwei bis drei, ganz besonders bevorzugt zwei, neutralen Wäsche(n) mit Wasser gewaschen und anschließend von der wässrigen Phase durch Phasentrennung; getrennt bei mehreren Wäschen nach jeder einzelnen Wäsche (Schritt d(iii)). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt. Bevorzugt ist eine Verfahrensweise, bei der in der letzten neutralen Wäsche eine Elektrophorese eingesetzt wird (siehe WO 2012/013678 A2).

Das gewaschene Nitrobenzol wird schließlich von gelöstem Wasser, nicht umgesetztem Benzol und ggf. organischen Verunreinigungen durch weitere Aufarbeitung befreit (Schritt d(iv)). Diese Aufarbeitung erfolgt bevorzugt durch Destillation, wobei über Kopf die Brüden Wasser und Benzol und ggf. organische Verunreinigungen ausgetrieben werden. Die Brüden werden gekühlt und in einen Trennbehälter gefahren. In der unteren Phase setzt sich Wasser ab, das abgetrennt wird. In der oberen Phase befinden sich Benzol und Leichtsieder, die als Rückbenzol (d.2) wieder der Reaktion zugeführt werden. Bevorzugt wird als Destillationsapparat eine Rektifizierkolonne eingesetzt. Das Sumpfprodukt der Destillation wird, ggf. nach einer weiteren Destillation, in welcher Nitrobenzol *als Destillat* (also als Kopf- oder Seitenstromprodukt) erhalten wird, als Rein-Nitrobenzol (d.1) weiteren Anwendungen (wie der Hydrierung zu Anilin) zugeführt.

Erfindungswesentlich ist nun, dass der aufkonzentrierten wässrigen, Schwefelsäure-haltigen Phase (c.1) (Kreislaufschwefelsäure) mindestens 1 Minute, bevorzugt 1 Minute bis 10 Minuten, besonders bevorzugt 3 Minuten bis 7 Minuten bevor diese mit dem Salpetersäurestrom (a.3) in Kontakt tritt, ein Oxidationsmittel (c.2) in einem Maß zugesetzt wird, dass sich eine Konzentration an Oxidationsmittel (c.2) von 10 ppm bis 5.000 ppm, bevorzugt 50 ppm bis 2.000 ppm und besonders bevorzugt 100 ppm bis 1.000 ppm, jeweils bezogen auf die Gesamtmasse der in Schritt a) zurückzuführenden aufkonzentrierten wässrigen, Schwefelsäure-haltigen Phase (c.1), einstellt. Der Mindestzeitraum von 1 Minute ist wesentlich, weil bei geringerer Verweilzeit der Salpetersäure in der Schwefelsäure die positiven Effekte (Vermeidung von Niederschlägen) nicht mehr auftreten. Es genügt zum Beispiel nicht, das Oxidationsmittel (c.2) direkt vor der Nitrierreaktion auf die Saugseite der Schwefelsäurekreislaufpumpe zu geben, denn in diesem Fall dauert es in der Regel weniger als 30 Sekunden, bis die Kreislaufschwefelsäure (c.1) in den Nitrierreaktor eintritt. Bevorzugte Oxidationsmittel sind Salpetersäure (c.2.1), salpetrige Säure (c.2.2) und/oder Nitrosylsulfonsäure (c.2.3).

In einer bevorzugten Ausführungsform des erfindungsgemäßen Verfahrens wird die Kreislaufschwefelsäure (c.1) in einem Schwefelsäurevorlagetank für ihren Einsatz in Schritt a) bereitgehalten, und die Zugabe des Oxidationsmittels (c.2), bevorzugt der Salpetersäure (c.2.1), erfolgt in die Schwefelsäure führende Rohrleitung zwischen Verdampfungsapparat (dem sog. "Blitzverdampfer") und dem Schwefelsäurevorlagetank, bevorzugt unter intensiver Vermischung.

Wird die Kreislaufschwefelsäure (c.1) erfindungsgemäß mit einem Oxidationsmittel versetzt, bevor sie in Schritt a) zurückgeführt und als Bestandteil von (a.2) eingesetzt wird, ergeben sich die folgenden Vorteile für die Herstellung von Nitrobenzol:
i) Es treten nur noch Spuren von schwarzem Niederschlag im Rohnitrobenzolwärmeaustauscher und im Phasentrennapparat auf, so dass die Anlagenverfügbarkeit stark verbessert ist.
ii) Anbackungen am Ausgang des Phasentrennapparates zum Rohnitrobenzoltank treten nur noch sehr geringfügig auf.
iii) Instandhaltungskosten (Reinigung der Apparate) werden verringert.
iv) Verbrennungskosten für die Rückstandsbeseitigung fallen nicht an.

Diese erfindungsgemäße Vorgehensweise ist für den Fachmann auch deshalb nicht naheliegend, da er annehmen muss, dass eine Behandlung der Kreislaufschwefelsäure nach dem Blitzverdampfer keinen weiteren positiven Effekt bringt, weil im Bereich des Ausganges des Blitzverdampfers, in den Rohrleitungen zum Schwefelsäurevorlagetank und im Schwefelsäurevorlagetank selbst keinerlei schwarzer Niederschlag zu finden ist.

### Beispiele

### Beispiele 1 und 2: Vorversuche im Labormaßstab

### Beispiel 1:

Lösungen von 50 g Kreislaufschwefelsäure, die aus dem Schwefelsäurevorlagetank einer adiabat betriebenen Nitrobenzolanlage stammen, die mit 0,88 g Nitrobenzol versetzt waren, wurden in einer verschlossenen Glasflasche unter Schütteln über verschieden lange Zeitspannen bei 150 °C getempert. Nach Beendigung der Handversuche wurden die jeweiligen Lösungen durch einen Glasfaserfilter mit einer Porengröße von 0,6 µm filtriert. Die Filter inklusive Niederschlag wurden für 21 Stunden bei 55 °C und einem Druck von 100 mbar getrocknet. Die auf dem Filter verbliebenen schwarzen Niederschläge wurden gewogen (siehe Tabelle 1).

**Tabelle 1: Abhängigkeit der Niederschlagsmenge von der Dauer des Temperierens**

| | | | | | | |
|---|---|---|---|---|---|---|
| Zeitspanne [h] | 0 | 12 | 30 | 52 | 76 | 102 |
| Niederschlag [g] | 0 | 0,09 | 0,23 | 0,34 | 0.36 | 0,37 |

Die Menge an schwarzem Niederschlag nahm wie erwartet mit der Dauer des Handversuches zu. Die Elementaranalyse auf C (Kohlenstoff), H (Wasserstoff), N (Stickstoff) und O (Sauerstoff) fiel ähnlich aus wie für den schwarzen Niederschlag aus Beispiel 3 (C: 56,3 %; H: 3,0 %; N: 4,3 %; O: 34,2 %; S: 2,0 %). Eine IR-Analyse zeigte die Anwesenheit von Aminen. Eine Dünnschichtchromatographie in Kombination mit UV-Detektion und chemischem Nachweis mit Pinacryptolgelb deutete auf Sulfonsäure hin. Eine Dünnschichtchromatographie in Kombination mit Diazotierung mit N-(1-Naphthyl)ethylendiamindihydrochlorid (Bratton-Marshall-Reagenz) bestätigte die Anwesenheit von Aminen. Die zwei größten Unbekannten aus der Dünnschichtchromatographie konnten isoliert und aufgrund ihrer chemischen Lage im Chromatogramm nach Abgleich mit einer Datenbank als Aminobenzolsulfonsäure und 4-Aminophenol identifiziert werden. Die Vermutung liegt nahe, dass der obige Niederschlag dem im Artikel *"Researches on the action of sulfuric acid on certain nitrocarbocyclic compounds. I. The action of nitrobenzene"* von M. L. Crossley und C. B. Ogilvie in J. Am. Chem. Soc., 1917, Vol 39 [1], S. 117 - 122, beschriebenen schwarzen Niederschlag entsprechen könnte.

### Beispiel 2:

Die Versuche wurden wie unter Beispiel 1 beschrieben durchgeführt. Die Versuchsdauer betrug jeweils 23 Stunden. Den Lösungen wurden verschiedene Mengen an unterschiedlichen Oxidationsmitteln zugesetzt. Es wurde wieder die Menge an schwarzem Niederschlag ausgewogen. Die Farbe der Lösungen und das Entstehen von braunen Stickoxidgasen wurden qualitativ analysiert. Das Entstehen von Dinitrobenzol in den Lösungen wurde quantitativ analysiert (siehe Tabelle 2).

**Tabelle 2: Einfluss von der Art und Menge an Oxidationsmittel auf Niederschlagsmenge, NOx-Entwicklung, Farbe der Lösung und Dinitrobenzolgehalt der Lösung**

| Oxidationsmittel | Vergleich: ohne Oxidations mittel | Salpetersäure | | Salpetrige Säure^{[a]} | | Nitrosylsulfonsäure | |
|---|---|---|---|---|---|---|---|
| Menge [ppm] | 0 | 1.000 | 5.000 | 1.000 | 5.000 | 1.000 | 5.000 |
| Niederschlag [g] | 0,2 | 0,05 | 0,01 | 0,15 | 0,02 | 0,02 | 0,01 |
| Farbe | schwarz | schwarz | hellgelb | schwarz | hellgelb | schwarz | hellgelb |
| Dinitrobenzol [ppm] | 2 | 175 | 2530 | nicht bestimmt | 52 | nicht bestimmt | 10 |
| Stickoxide | nein | nein | ja | ja | ja | nein | nein |

| | | | | | | | |
|---|---|---|---|---|---|---|---|
| [a] *In situ* aus Natriumnitrit hergestellt. | | | | | | | |

Man sieht, dass durch Einsatz von Oxidationsmittel die Entstehung von schwarzem Niederschlag stark reduziert wird. Bei Einsatz von erhöhten Mengen an Salpetersäure und bei salpetriger Säure entstehen braune Stickoxidgase. Erhöhte Mengen an Dinitrobenzol entstehen vor allen Dingen bei Verwendung an erhöhten Mengen von Salpetersäure als Oxidationsmittel. Die deutliche Reduzierung des Niederschlags ist ein Vorteil, der das Auftreten von Stickoxidgasen und die Bildung von Dinitrobenzol mehr als kompensiert.

### Beispiele 3 bis 7: Gegenüberstellung von erfindungsgemäßer Vorgehensweise mit der Vorgehensweise des Standes der Technik unter Betriebsbedingungen

### Allgemeine Bedingungen für die Herstellung von Nitrobenzol

Einem Reaktor wird ein Schwefelsäurestrom, in den zuerst ein Salpetersäurestrom und anschließend ein vereinter Frischbenzol- und Rückbenzolstrom eindosiert wurden, zugeführt. Die Verweilzeit zwischen der Zugabe von Salpetersäure zur Schwefelsäure und dem Eintritt der Nitrierlösung in den Reaktor ist kleiner als 10 Sekunden. Es werden 6 % Rückbenzol als Überschussbenzol gefahren. Nach vollständiger Umsetzung der Salpetersäure mit dem Benzol zu Nitrobenzol unter adiabater Reaktionsführung wird das nunmehr ca. 130 °C heiße Reaktionsprodukt einem Phasentrennapparat zugeführt, in dem das Reaktionsprodukt in eine organische Phase (= Roh-Nitrobenzol, enthaltend neben Nitrobenzol noch Benzol) und eine wässrige Phase (= Absäure, enthaltend neben Schwefelsäure noch geringe Anteile an Nitrobenzol und Benzol) zerfällt. Die wässrige, hauptsächlich Schwefelsäure umfassende Phase wird in einem Verdampfer durch plötzliche Druckerniedrigung einer Blitzverdampfung von Wasser unterzogen und auf diese Weise aufkonzentriert. Die aufkonzentrierte Schwefelsäure wird in einem Schwefelsäurevorlagetank zur erneuten Verwendung in der Nitrierung zwischengelagert. Nach dem Abtrennen im Phasentrennapparat wird das Rohnitrobenzol in einer Rohnitrobenzolkühlung auf ca. 40 °C bis 50 °C abgekühlt und zur Aufarbeitung einer Wäsche zugeführt. Der so erhaltene von Nitrophenolen und Salzen weitgehend befreite Strom des gereinigten Nitrobenzols wird wieder aufgeheizt und in einer Rektifikationskolonne von Wasser und Benzol, welche über Kopf abgetrennt werden, befreit, wodurch im Sumpf der Kolonne getrocknetes Rein-Nitrobenzol erhalten wird. Das kondensierte Kopfprodukt der Rektifikationskolonne wird einem Phasentrennapparat zugeführt, in dem das Kopfprodukt in eine organische Phase (enthaltend Benzol) und eine wässrige Phase zerfällt. Das Benzol wird in einem Puffertank zwischengelagert und von dort als Rückbenzol, wie oben schon beschrieben, zur Reaktion in den Zulauf des Reaktors zurückgefahren.

### Beispiele 3 (Vergleichsbeispiel)

Die Herstellung von Nitrobenzol wurde, wie in den allgemeinen Bedingungen für die Herstellung von Nitrobenzol beschrieben, durchgeführt. Im Phasentrennapparat sammelten sich in der Phasentrennschicht schwarze, schwebende Feststoffpartikel, und am Ausgang des Trennapparates zum Rohnitrobenzoltank gab es schwarze Anbackungen, die zweimal pro Jahr aus dem Apparat entfernt werden mussten, um Betriebsstörungen zu vermeiden. Der Wärmeaustauscher zur Rohnitrobenzolkühlung im Ablauf des Phasentrennapparates musste zehnmal im Jahr geöffnet werden, um schwarzen Niederschlag, der den Ablauf des Rohnitrobenzols verhinderte, zu entfernen.

**Tabelle 3: Analyse des schwarzen Niederschlages:**

| Element | C | H | N | O | S | Si | Metalle |
|---|---|---|---|---|---|---|---|
| Menge^{[a]} (%) | 58,5 | 3,6 | 4,2 | 31,5 | 1,3 | 0,7 | 0,2 |

**Tabelle 4: Im schwarzen Niederschlag nachgewiesene Metalle:**

| Metall | Al | Cr | Cu | Fe | Pd | Pt | Ta | Ti |
|---|---|---|---|---|---|---|---|---|
| Menge^{[a]} (%) | 0,010 | 0,029 | 0,006 | 0,011 | 0,059 | 0,063 | 0,007 | 0,008 |

| | | | | | | | | |
|---|---|---|---|---|---|---|---|---|
| [a] Massenanteil, bezogen auf die Masse des schwarzen Niederschlags. | | | | | | | | |

C (Kohlenstoff), H (Wasserstoff), N (Stickstoff), und O (Sauerstoff) wurden per Elementaranalyse bestimmt. Si (Silizium), S (Schwefel) und Metalle wurden per Röntgenfluoreszenz bestimmt.

### Beispiel 4 (Vergleichsbeispiel)

Die Herstellung von Nitrobenzol wurde, mit folgender ergänzender Maßnahme, wie in den allgemeinen Bedingungen für die Herstellung von Nitrobenzol beschrieben, durchgeführt:
Auf die Saugseite der Schwefelsäurekreislaufpumpe wurden 500 ppm an Salpetersäure gegeben, um die Pumpe zu passivieren. Die so behandelte Schwefelsäure hatte eine Verweilzeit von ca. 3 Sekunden bis zum Reaktionseintritt. Zwischen der Schwefelsäurepumpe und dem Reaktionseintritt wurde zuerst Salpetersäure und dann direkt am Reaktoreintritt Benzol in den Schwefelsäurestrom eindosiert. Die Problematik mit dem schwarzen Niederschlag stellte sich genauso, wie in Beispiel 1 beschrieben, dar. Die Analytik zeigte im Rahmen der Messgenauigkeit eine chemisch identische Zusammensetzung wie in Beispiel 1 an.

### Beispiel 5 (erfindungsgemäß)

Die Herstellung von Nitrobenzol wurde, mit Ausnahme folgender ergänzender Maßnahme, wie in den allgemeinen Bedingungen für die Herstellung von Nitrobenzol beschrieben, durchgeführt:
In den Schwefelsäurevorlagetank wurde kontinuierlich Salpetersäure (c.2.1) so eingeleitet, dass sich ein errechneter Massenanteil an Salpetersäure, bezogen auf die Gesamtmasse von Schwefelsäure und Salpetersäure, von 1.000 ppm, einstellte. Die so behandelte Schwefelsäure (c.1) hatte eine Verweilzeit von ca. 5 min bis zum Eintritt in die Nitrierreaktion (Schritt a)). Es wurden nur Spuren an schwarzem Niederschlag durch ein Schauglas im Phasentrennapparat beobachtet. Der Wärmeaustauscher zur Rohnitrobenzolkühlung im Ablauf des Phasentrennapparates musste nur einmal im Jahr geöffnet werden, um schwarzen Niederschlag, der den Ablauf des Rohnitrobenzols verhindert, zu entfernen.

### Beispiel 6 (erfindungsgemäß)

Die Herstellung von Nitrobenzol wurde, mit folgender ergänzender Maßnahme, wie in den allgemeinen Bedingungen für die Herstellung von Nitrobenzol beschrieben, durchgeführt:

In den Schwefelsäurevorlagetank wurde kontinuierlich Salpetersäure (c.2.1) so eingeleitet, dass sich ein errechneter Massenanteil an Salpetersäure, bezogen auf die Gesamtmasse von Schwefelsäure und Salpetersäure, von 200 ppm, einstellte. Die so behandelte Schwefelsäure (c.1) hatte eine Verweilzeit von ca. 5 min bis zum Eintritt in die Nitrierreaktion (Schritt a)). Es wurden nur Spuren an schwarzem Niederschlag durch ein Schauglas im Phasentrennapparat beobachtet. Der Wärmeaustauscher zur Rohnitrobenzolkühlung im Ablauf des Phasentrennapparates musste nur einmal im Jahr geöffnet werden, um schwarzen Niederschlag, der den Ablauf des Rohnitrobenzols verhindert, zu entfernen.

### Beispiel 7 (erfindungsgemäß)

Die Herstellung von Nitrobenzol wurde, mit folgender ergänzender Maßnahme, wie in den allgemeinen Bedingungen für die Herstellung von Nitrobenzol beschrieben, durchgeführt:
In den Schwefelsäurevorlagetank wurde kontinuierlich Nitrosylsulfonsäure (c.2.3) so eingeleitet, dass sich ein errechneter Massenanteil an Nitrosylsulfonsäure, bezogen auf die Gesamtmasse von Schwefelsäure und Salpetersäure, von 40 ppm, einstellte. Es wurden nur Spuren an schwarzem Niederschlag durch ein Schauglas im Phasentrennapparat beobachtet. Der Wärmeaustauscher zur Rohnitrobenzolkühlung im Ablauf des Phasentrennapparates musste nur einmal im Jahr geöffnet werden, um schwarzen Niederschlag, der den Ablauf des Rohnitrobenzols verhindert, zu entfernen.

## Patentansprüche

1. Kontinuierlich betriebenes adiabates Verfahren zur Herstellung von Nitrobenzol durch Nitrierung von Benzol, bei dem
a) ein Benzol-haltiger Strom (a.1) in einem Reaktor mit Schwefelsäure (a.2) und Salpetersäure (a.3) unter adiabaten Bedingungen umgesetzt wird, wobei Benzol in einem stöchiometrischen Überschuss bezogen auf Salpetersäure (a.3) eingesetzt wird,
b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, wobei die Phase (c.1) in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird,
**dadurch gekennzeichnet, dass**
der aufkonzentrierten wässrigen, Schwefelsäure-haltigen Phase (c.1) mindestens 1 Minute bevor diese mit dem Salpetersäurestrom (a.3) in Kontakt tritt, ein Oxidationsmittel (c.2) ausgewählt aus der Gruppe bestehend aus Salpetersäure (c.2.1), salpetrige Säure (c.2.2), Nitrosylsulfonsäure (c.2.3) und einem Gemisch von mindestens zwei dieser Oxidationsmittel in einem Maß zugesetzt wird, dass sich eine Konzentration des Oxidationsmittels (c.2) von 10 ppm bis 5.000 ppm, bezogen auf die Gesamtmasse der in Schritt a) zurückzuführenden aufkonzentrierten wässrigen, Schwefelsäure-haltigen Phase (c.1), einstellt.

2. Verfahren nach Anspruch 1, bei dem das Oxidationsmittel (c.2) 1 Minute bis 10 Minuten, bevor die Schwefelsäure-haltige Phase (c.1) mit dem Salpetersäurestrom (a.3) in Kontakt tritt, zugesetzt wird.

3. Verfahren nach Anspruch 1, bei dem das Oxidationsmittel (c.2) 3 Minuten bis 7 Minuten, bevor die Schwefelsäure-haltige Phase (c.1) mit dem Salpetersäurestrom (a.3) in Kontakt tritt, zugesetzt wird.

4. Verfahren nach einem der Ansprüche 1 bis 3, bei dem eine Konzentration des Oxidationsmittels (c.2) von 50 ppm bis 2.000 ppm, bezogen auf die Gesamtmasse der in Schritt a) zurückzuführenden aufkonzentrierten, wässrigen, Schwefelsäure-haltigen Phase (c.1), eingestellt wird.

5. Verfahren nach einem der Ansprüche 1 bis 3, bei dem eine Konzentration des Oxidationsmittels (c.2) von 100 ppm bis 1.000 ppm, bezogen auf die Gesamtmasse der in Schritt a) zurückzuführenden aufkonzentrierten wässrigen, Schwefelsäure-haltigen Phase (c.1), eingestellt wird.

6. Verfahren nach einem der Ansprüche 1 bis 5, bei dem die Schwefelsäure-haltige Phase (c.1) in einem Schwefelsäurevorlagetank für ihren Einsatz in Schritt a) bereitgehalten wird, und bei dem die Zugabe des Oxidationsmittels (c.2) in eine Rohrleitung von dem in Schritt c) eingesetzten Verdampfungsapparat zu dem Schwefelsäurevorlagetank erfolgt.

## Claims

1. Continuously operated adiabatic process for the preparation of nitrobenzene by nitration of benzene, in which
a) a benzene-containing stream (a.1) is reacted in a reactor with sulfuric acid (a.2) and nitric acid (a.3) under adiabatic conditions, wherein benzene is employed in a stoichiometric excess, based on nitric acid (a.3),
b) the process product obtained in step a) is separated in a phase separation apparatus into an aqueous phase (b.1) comprising sulfuric acid and an organic phase (b.2) comprising nitrobenzene,
c) the aqueous phase (b.1) obtained in step b) is concentrated by evaporation of water to give an aqueous phase (c.1) having an increased sulfuric acid concentration compared with (b.1), wherein phase (c.1) is returned into step a) and is used as a component of (a.2),
d) the organic phase (b.2) obtained in step b) is worked up to give pure nitrobenzene (d.1),
**characterized in that**
an oxidizing agent (c.2) chosen from the group consisting of nitric acid (c.2.1), nitrous acid (c.2.2), nitrosylsulfonic acid (c.2.3) and a mixture of at least two of these oxidizing agents is added to the concentrated aqueous phase (c.1) comprising sulfuric acid at least 1 minute before this comes into contact with the nitric acid stream (a.3), to an extent such that a concentration of the oxidizing agent (c.2) of from 10 ppm to 5,000 ppm, based on the total weight of the concentrated aqueous phase (c.1) comprising sulfuric acid to be returned into step a), is established.

2. Process according to claim 1, in which the oxidizing agent (c.2) is added 1 minute to 10 minutes before the phase (c.1) comprising sulfuric acid comes into contact with the nitric acid stream (a.3).

3. Process according to claim 1, in which the oxidizing agent (c.2) is added 3 minutes to 7 minutes before the phase (c.1) comprising sulfuric acid comes into contact with the nitric acid stream (a.3).

4. Process according to one of claims 1 to 3, in which a concentration of the oxidizing agent (c.2) of from 50 ppm to 2,000 ppm, based on the total weight of the concentrated aqueous phase (c.1) comprising sulfuric acid to be recycled into step a), is established.

5. Process according to one of claims 1 to 3, in which a concentration of the oxidizing agent (c.2) of from 100 ppm to 1,000 ppm, based on the total weight of the concentrated aqueous phase (c.1) comprising sulfuric acid to be recycled into step a), is established.

6. Process according to one of claims 1 to 5, in which the phase (c.1) comprising sulfuric acid is held ready in a sulfuric acid reservoir tank for its use in step a), and in which the addition of the oxidizing agent (c.2) takes place in a pipeline from the evaporation apparatus employed in step c) to the sulfuric acid reservoir tank.

## Revendications

1. Procédé adiabatique exploité en continu pour la fabrication de nitrobenzène par nitration de benzène, selon lequel
a) un courant contenant du benzène (a.1) est mis en réaction dans un réacteur avec de l'acide sulfurique (a.2) et de l'acide nitrique (a.3) dans des conditions adiabatiques, le benzène étant utilisé en un excès stoechiométrique par rapport à l'acide nitrique (a.3),
b) le produit de procédé obtenu à l'étape a) est séparé dans un appareil de séparation de phases en une phase aqueuse comprenant de l'acide sulfurique (b.1) et une phase organique comprenant du nitrobenzène (b.2),
c) la phase aqueuse (b.1) obtenue à l'étape b) est concentrée par évaporation d'eau en une phase aqueuse (c.1) ayant une concentration en acide sulfurique augmentée par rapport à (b.1), la phase (c.1) étant recyclée dans l'étape a) et utilisée en tant que constituant de (a.2),
d) la phase organique (b.2) obtenue à l'étape b) est traitée pour obtenir du nitrobenzène pur (d.1),
**caractérisé en ce que**
un oxydant (c.2) choisi dans le groupe constitué par l'acide nitrique (c.2.1), l'acide nitreux (c.2.2), l'acide nitrosylsulfonique (c.2.3) et un mélange d'au moins deux de ces oxydants est ajouté à la phase aqueuse concentrée contenant de l'acide sulfurique (c.1) au moins 1 minute avant la mise en contact de celle-ci avec le courant d'acide nitrique (a.3) en une mesure telle qu'une concentration de l'oxydant (c.2) de 10 ppm à 5 000 ppm, par rapport à la masse totale de la phase aqueuse concentrée contenant de l'acide sulfurique (c.1) recyclée dans l'étape a), s'ajuste.

2. Procédé selon la revendication 1, selon lequel l'oxydant (c.2) est ajouté 1 minute à 10 minutes avant la mise en contact de la phase contenant de l'acide sulfurique (c.1) avec le courant d'acide nitrique (a.3).

3. Procédé selon la revendication 1, selon lequel l'oxydant (c.2) est ajouté 3 minutes à 7 minutes avant la mise en contact de la phase contenant de l'acide sulfurique (c.1) avec le courant d'acide nitrique (a.3).

4. Procédé selon l'une quelconque des revendications 1 à 3, dans lequel une concentration de l'oxydant (c.2) de 50 ppm à 2 000 ppm, par rapport à la masse totale de la phase aqueuse concentrée contenant de l'acide sulfurique (c.1) recyclée dans l'étape a), est ajustée.

5. Procédé selon l'une quelconque des revendications 1 à 3, dans lequel une concentration de l'oxydant (c.2) de 100 ppm à 1 000 ppm, par rapport à la masse totale de la phase aqueuse concentrée contenant de l'acide sulfurique (c.1) recyclée dans l'étape a), est ajustée.

6. Procédé selon l'une quelconque des revendications 1 à 5, dans lequel la phase contenant de l'acide sulfurique (c.1) est tenue prête dans une cuve de collecte d'acide sulfurique pour son utilisation dans l'étape a), et dans lequel l'ajout de l'oxydant (c.2) a lieu dans une canalisation depuis l'appareil d'évaporation utilisé à l'étape c) vers la cuve de collecte d'acide sulfurique.
